(19) Europäisches Patentamt
European Patent Office
Office européen des brevets

(11) EP 0 662 326 B1

(12) EUROPÄISCHE PATENTSCHRIFT

(45) Veröffentlichungstag und Bekanntmachung des Hinweises auf die Patenterteilung:
**28.11.2001 Patentblatt 2001/48**

(51) Int Cl.[7]: **A61K 38/08**
// (A61K38/08, 31:55, 31:495, A61P33:10)

(21) Anmeldenummer: **94120772.2**

(22) Anmeldetag: **27.12.1994**

# (54) Endoparasitizide Mittel die Praziquantel und Epsiprantel enthalten

Endoparasitical agents containing praziquantel and epsiprantel

Agents antiparasiticides contenant du praziquantel et de l'epsiprantel

(84) Benannte Vertragsstaaten:
**AT BE CH DE DK ES FR GB GR IE IT LI NL PT SE**

(30) Priorität: **11.01.1994 DE 4400464**

(43) Veröffentlichungstag der Anmeldung:
**12.07.1995 Patentblatt 1995/28**

(73) Patentinhaber: **BAYER AG**
**51368 Leverkusen (DE)**

(72) Erfinder:
- **Mencke, Norbert, Dr.**
  **D-51381 Leverkusen (DE)**
- **Harder, Achim, Dr.**
  **D-51109 Köln (DE)**
- **Jeschke, Peter, Dr.**
  **D-51373 Leverkusen (DE)**

(56) Entgegenhaltungen:
**EP-A- 0 382 173**
**EP-A- 0 626 375**
**EP-A- 0 626 376**
**WO-A-92/08468**
**WO-A-93/25543**
**WO-A-94/19334**
**GB-A- 2 252 730**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

EP 0 662 326 B1

## Beschreibung

Die vorliegende Erfindung betrifft die Verwendung von Praziquantel und Epsiprantel zur Steigerung der endoparasitiziden Wirkung von cyclischen Depsipeptiden in endoparasitiziden Mitteln.
Praziquantel 2-(Cyclohexylcarbonyl)-1,2,3,6,7,11b-hexahydro-4H-pyrazino[2,1-a]isoquinolin-4-on und seine Wirkung gegen Endoparasiten ist bekannt aus GB-Pat 1 441 554.
Epsiprantel 2-(Cyclohexylcarbonyl)-2,3,6,7,8,12b-hexahydro-pyrazino[2,1-a][2]-benzazepin-4(1H)-on und seine Wirkung gegen Endoparasiten ist bekannt aus EP-OS 134 984, EP-OS 185 012.
Ein cyclisches Depsipeptid PF1022 und seine Wirkung gegen Endoparasiten ist bekannt aus EP-OS 382 173.
Weitere cyclische Depsipeptide und ihre endoparasitizide Wirkung sind Gegenstand nicht vorveröffentlichter Patentanmeldungen (Deutsche Patentanmeldungen P 4 317 432.9; P 4 317 457.4; P 4 317 458.2).
Gegenstand der vorliegenden Erfindung sind:
Endoparasitizide Mittel, die Praziquantel oder Epsiprantel zusammen mit cyclischen Depsipeptiden der Formel

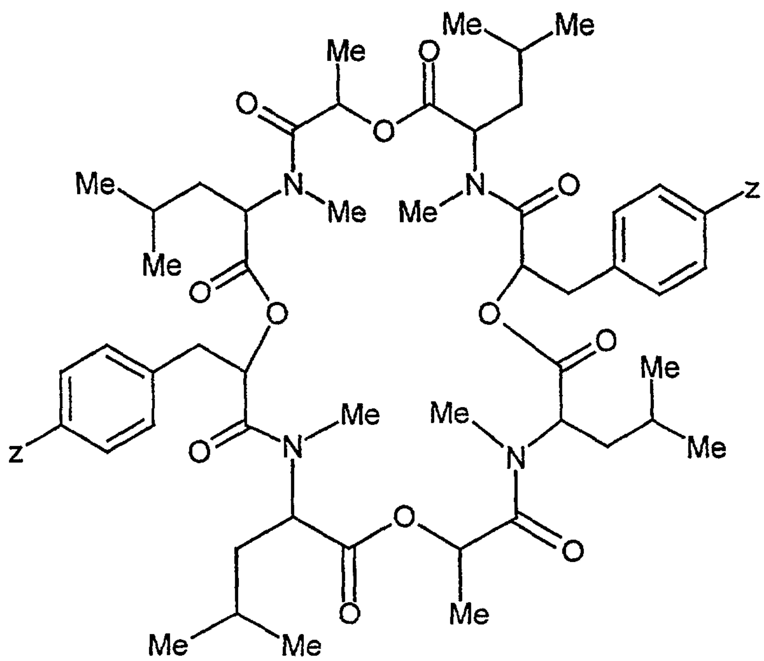

in welcher

Z für Wasserstoff (PF 1022A), N-Morpholino (Bis-morpholino-derivat, PF 1022-21), Nitro, Amino oder Dimethylamino steht,

bestehend aus Aminosäuren und Hydroxycarbonsäuren als Ringbausteinen und mit 24 Ringatomen enthalten.
Gegenstand der Erfindung ist ferner die Verwendung von Praziquantel oder Epsiprantel zusammen mit cyclischen Depsipeptiden der Formel

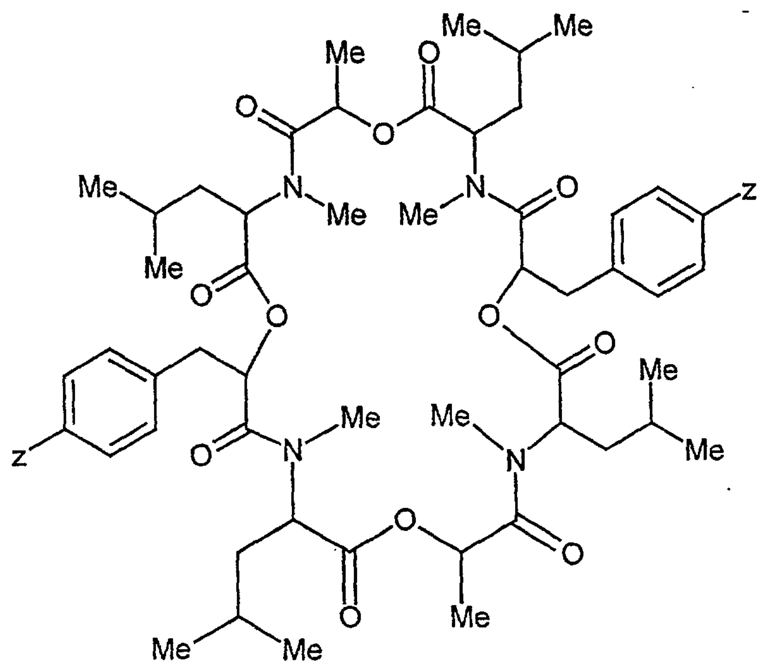

in welcher

Z für Wasserstoff (PF 1022A), N-Morpholino (Bis-morpholino-derivat, PF 1022-21), Nitro, Amino oder Dimethylamino steht,

bestehend aus Aminosäuren und Hydroxycarbonsäuren als Ringbausteinen und mit 24 Ringatomen zur Herstellung von endoparasitiziden Mitteln.

Im Sinne der vorliegenden Erfindung können alle Verbindungen der allgemeinen Formel (I), die in optisch aktiven, stereoisomeren Formen oder als racemische Gemische vorliegen können, verwendet werden. Vorzugsweise werden jedoch die optisch aktiven, stereoisomeren Formen der Verbindungen der allgemeinen Formel (I) erfindungsgemäß verwendet.

Als Depsipeptid sei die aus EP-OS 382 173 bekannte Verbindung PF 1022 der folgenden Formel genannt:

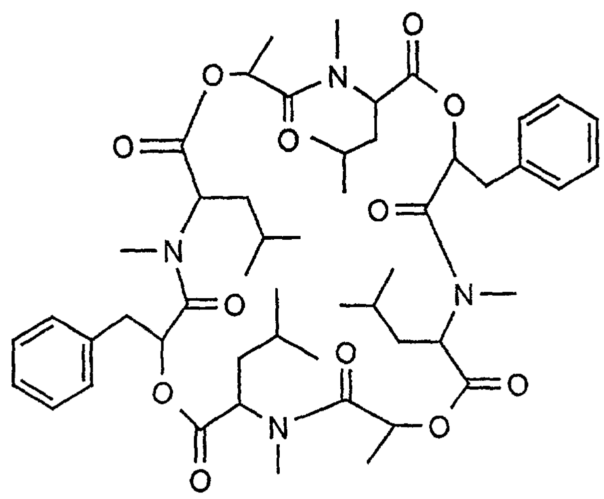

Außerdem seien als Depsipeptide die aus der PCT-Anmeldung WO 93/19053 bekannten Verbindungen genannt.

Insbesondere seien aus PCT-Anmeldung WO 93/19053 die Verbindungen der folgenden Formel genannt:

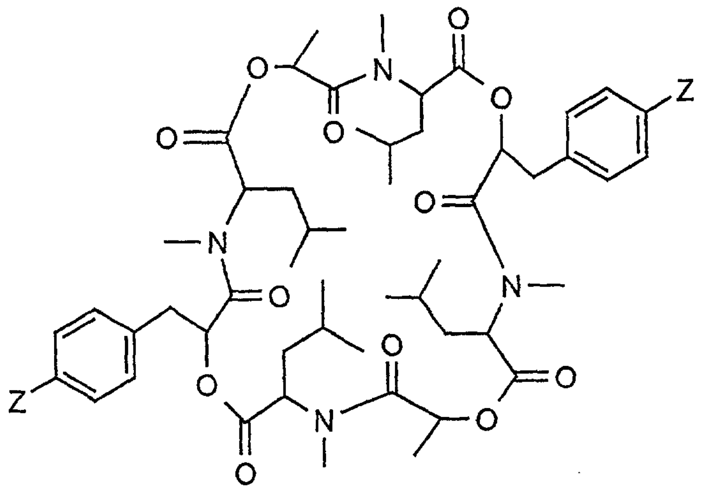

in welcher

Z für N-Morpholinyl, Nitro, Amino, Mono- oder Dimethylamino steht.

Zu den cyclischen Depsipeptiden mit 24 Ringatomen zählen Verbindungen der allgemeinen Formel (Ia)

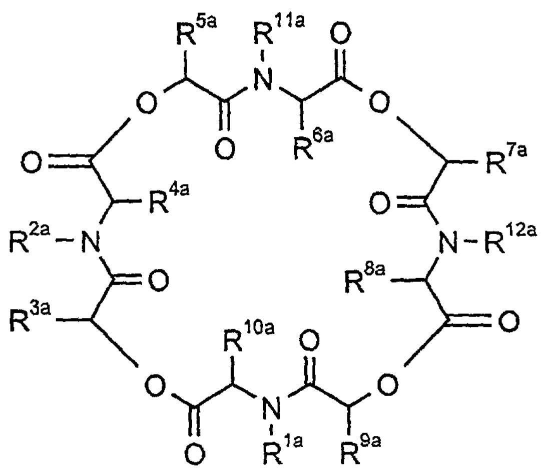

(Ia)

in welcher

| | |
|---|---|
| $R^{1a}$, $R^{2a}$, $R^{11a}$ und $R^{12a}$ | unabhängig voneinander für $C_{1-8}$-Alkyl, $C_{1-8}$-Halogenalkyl, $C_{3-6}$-Cycloalkyl, Aralkyl, Aryl stehen, |
| $R^{3a}$, $R^{5a}$, $R^{7a}$, $R^{9a}$ | unabhängig voneinander für Wasserstoff oder geradkettiges oder verzweigtes $C_{1-8}$-Alkyl steht, das gegebenenfalls durch Hydroxy, $C_{1-4}$-Alkoxy, Carboxy, |

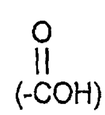,

Carboxamid,

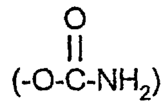,

Imidazolyl, Indolyl, Guanidino, -SH oder $C_{1-4}$-Alkylthio substituiert sein kann und ferner für Aryl oder Aralkyl die durch Halogen, Hydroxy, $C_{1-4}$-Alkyl, $C_{1-4}$-Alkoxy substituiert sein können, steht,

$R^{4a}$, $R^{6a}$, $R^{8a}$, $R^{10a}$ unabhängig voneinander für Wasserstoff, geradkettiges $C_{1-5}$-Alkyl, $C_{2-6}$-Alkenyl, $C_{3-7}$-Cycloalkyl, die gegebenenfalls durch Hydroxy, $C_{1-4}$-Alkoxy, Carboxy, Carboxamid, Imidazolyl, Indolyl, Guanidino, SH oder $C_{1-4}$-Alkylthio substituiert sein können, sowie für Aryl oder Aralkyl die durch Halogen, Hydroxy, $C_{1-4}$-Alkyl, $C_{1-4}$-Alkoxy substituiert sein können, stehen

sowie deren optische Isomere und Racemate.

Weiterhin seien Verbindungen der Formel (Ia) genannt, in welcher

$R^{1a}$, $R^{2a}$, $R^{11a}$ und $R^{12a}$ unabhängig voneinander für Methyl, Ethyl, Propyl, Isopropyl, n-, s-, t-Butyl oder Phenyl, das gegebenenfalls substituiert ist durch Halogen, $C_{1-4}$-Alkyl, OH, $C_{1-4}$-Alkoxy, sowie für Benzyl oder Phenylethyl stehen, die gegebenenfalls durch die bei Phenyl angegebenen Reste substituiert sein können;

$R^{3a}$ bis $R^{10a}$ die oben angegebene Bedeutung haben.

Weiterhin seien Verbindungen der Formel (Ia) genannt, in welcher

$R^{1a}$, $R^{2a}$, $R^{11a}$ und $R^{12a}$ unabhängig voneinander für Methyl, Ethyl, Propyl, Isopropyl oder n-, s-, t-Butyl stehen,

$R^{3a}$, $R^{5a}$, $R^{7a}$, $R^{9a}$ für Wasserstoff, geradkettiges oder verzweigtes $C_{1-8}$-Alkyl, insbesondere Methyl, Ethyl, Propyl, i-Propyl, n-, s-, t-Butyl, die gegebenenfalls durch C1-4-Alkoxy, insbesondere Methoxy, Ethoxy, Imidazolyl, Indolyl oder $C_{1-4}$-Alkylthio, insbesondere Methylthio, Ethylthio substituiert sein können, ferner für Phenyl, Benzyl oder Phenethyl stehen, die gegebenenfalls durch Halogen insbesondere Chlor substituiert sein können.

$R^{4a}$, $R^{6a}$, $R^{8a}$, $R^{10a}$ unabhängig voneinander für Wasserstoff, Methyl, Ethyl, n-Propyl, n-Butyl, Vinyl, Cyclohexyl, die gegebenenfalls durch Methoxy, Ethoxy, Imidazolyl, Indolyl, Methylthio, Ethylthio substituiert sein können sowie für Isopropyl, s-Butyl ferner für gegebenenfalls halogensubstituiertes Phenyl, Benzyl oder Phenylethyl stehen.

Die Verbindungen der Formel (Ia) können hergestellt werden, indem man offenkettige Octadepsipeptide der Formel (IIc)

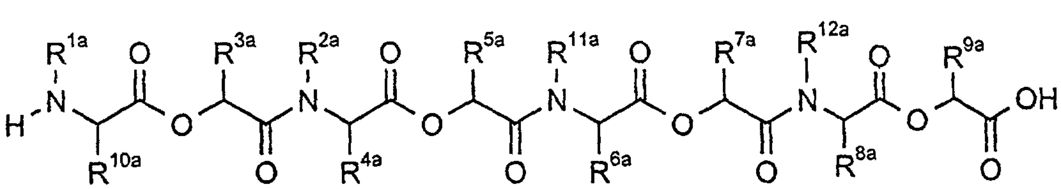

(IIc)

in welcher

$R^{1a}$ bis $R^{12a}$ die oben angegebene Bedeutung haben,

in Gegenwart eines Verdünnungsmittels und in Gegenwart eines Kupplungsreagenzes cyclisiert;

Als Kupplungsreagenzien eignen sich alle Verbindungen, die zur Knüpfung einer Amidbindung geeignet sind (vgl. z.B.: Houben-Weyl, Methoden der organischen Chemie, Band 15/2; Bodanszky et al., Peptide Synthesis 2nd ed. (Wiley / Sons, New York 1976).

Vorzugsweise kommen folgende Reagenzien und Methoden in Frage, Aktivestermethode mit Pentafluorphenol (Pfp), N-Hydroxysuccinimid, 1-Hydroxybenzotriazol, Kupplung mit Carbodiimiden, wie Dicyclohexylcarbodiimid oder N'-(3-Dimethylaminopropyl)-N-ethyl-carbodiimid (Ebc) sowie die gemischte Anhydrid-Methode oder die Kupplung mit Phosphoniumreagenzien, wie Benzotriazol-1-yl-oxy-tris(dimethylaminophosphonium)-hexafluorophosphat (BOP), Bis(2-oxo-3-oxazolidinyl)phosphoniumsäurechlorid (BOP-C1), oder mit Phosphonsäureesterreagentien, wie Cyanphosphonsäurediethylester (DEPC) und Diphenylphospharylazid (DPPA).

Besonders bevorzugt ist die Kupplung mit Bis(2-oxo-3-oxazolidinyl)-phosphoniumsäurechlorid (BOP-C1) und N'-(3-Dimethylaminopropyl)-N-ethylcarbodiimid (EDC) in Gegenwart von 1-Hydroxybenzotriazol (HOBt).

Die Umsetzung erfolgt bei Temperaturen von 0 - 150°C, bevorzugt bei 20 bis 100°C, besonders bevorzugt bei Raumtemperatur.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäure-methylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Die Verbindungen der Formeln (IIc) und die Kupplungsreagenzien werden im Verhältnis 1:1 bis 1:1,5 zueinander eingesetzt. Bevorzugt ist ein etwa äquimolares Verhältnis.

Nach erfolgter Umsetzung wird das Verdünnungsmittel abdestilliert und die Verbindungen der Formel (Ia) in üblicher Weise, z.B. chromatographisch, gereinigt.

Die offenkettigen Octadepsipeptide der Formel (IIc)

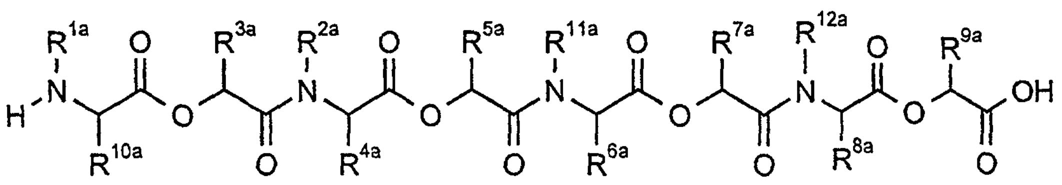

(IIc)

in welcher die Reste die oben angegebenen Bedeutungen haben werden erhalten, indem man Verbindungen der Formel (IIIa)

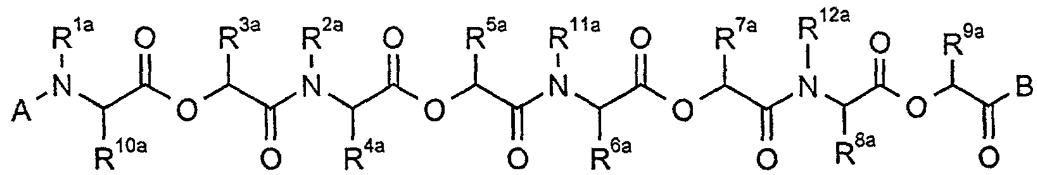

(IIIa)

in welcher

A für Benzyl und

B für OH steht und

$R^{1a}$ bis $R^{12a}$ die oben angegebene Bedeutung besitzen,

in Gegenwart eines Verdünnungsmittels und eines Katalysators hydrogenolysiert.

Die Verbindungen der Formel (IIIa)

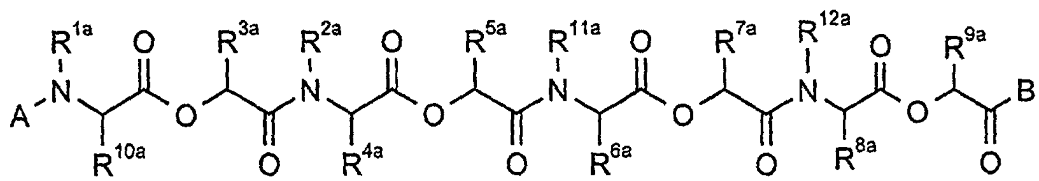

(IIIa)

in welcher die Reste die oben angegebene Bedeutung haben, werden erhalten, indem man Verbindungen der Formel (IVa)

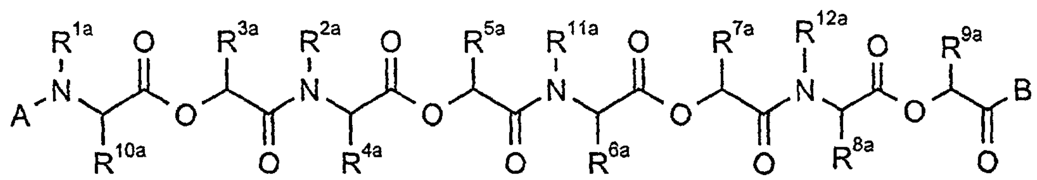

(IVa)

in welcher die Reste die oben angegebene Bedeutung haben hydrolysiert.

Verbindungen der Formel (IVa) sowie deren Stereoisomere, werden erhalten, indem man Tetradepsipeptide der Formel (Va)

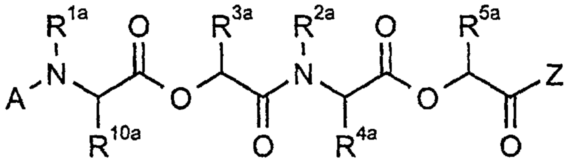 (Va)

in welcher

| | |
|---|---|
| A | für Benzyl und |
| Z | für OH steht sowie |
| $R^{1a}$, $R^{2a}$, $R^{3a}$, $R^{4a}$, $R^{5a}$ und $R^{10a}$ | die oben angegebene Bedeutung haben |

und Tetradepsipeptide der Formel (VIa)

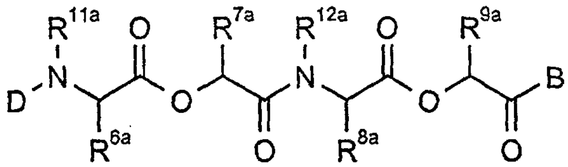 (VIa)

in welcher

| | |
|---|---|
| D | für Wasserstoff und |
| B | für tert.-Butoxy steht sowie |
| $R^{6a}$, $R^{7a}$, $R^{8a}$, $R^{9a}$, $R^{11a}$ und $R^{12a}$ | die oben angegebene Bedeutung haben, |

in Gegenwart eines Verdünnungsmittels und eines Kupplungsreagenzen kondensiert.

Tetradepsipeptide der Formel (Va) werden erhalten, indem man Tetradepsipeptide der Formel (VIIa)

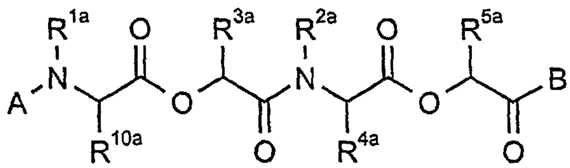 (VIIa)

in welcher

| | |
|---|---|
| A | für Benzyl und |
| B | für tert.-Butoxy steht sowie |
| $R^{1a}$, $R^{2a}$, $R^{3a}$, $R^{4a}$, $R^{5a}$ und $R^{10a}$ | die oben angegebene Bedeutung besitzen, |

in Gegenwart eines Verdünnungsmittels und einer Protonensäure verseift.

Tetradepsipeptide der Formel (VIa)

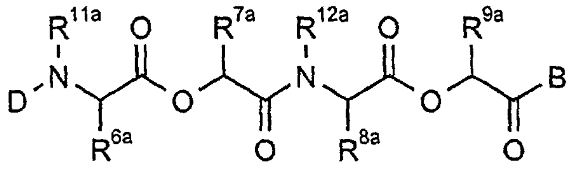

(VIa)

in welcher

D für Wasserstoff und

B für tert.-Butoxy steht und die übrigen Reste die oben angegebene Bedeutung haben,

werden erhalten, indem man Tetradepsipeptide der Formel (VIIa)

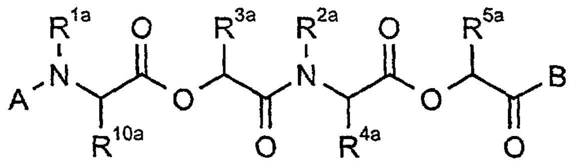

(VIIa)

in welcher

| | |
|---|---|
| A | für Benzyl und |
| B | für tert.-Butoxy steht sowie |
| $R^{1a}$, $R^{2a}$, $R^{3a}$, $R^{4a}$, $R^{5a}$ und $R^{10a}$ | die oben angegebene Bedeutung besitzen, |

in Gegenwart eines Verdünnungsmittels und eines Katalysators hydrogenolysiert.

Tetradepsipeptide der Formel (VIIa) werden erhalten, indem man Didepsipeptide der Formel (VIIIa)

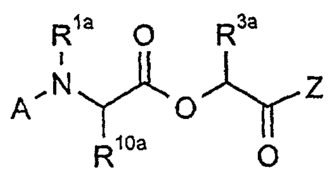

(VIIIa)

in welcher

| | |
|---|---|
| A | für Benzyl und |
| Z | für OH steht sowie |
| $R^{1a}$, $R^{3a}$ und $R^{10a}$ | die oben angegebene Bedeutung besitzen und |

Didepsipeptide der Formel (IXa)

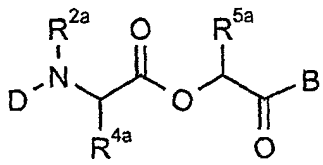

(IXa)

in welcher

D für Wasserstoff und

B für tert.-Butoxy steht sowie

$R^{2a}$, $R^{4a}$ und $R^{5a}$ die oben angegebene Bedeutung besitzen,

in einem Verdünnungsmittel in Gegenwart eines Kupplungsreagenzen kondensiert.

Die erfindungsgemäßen Mittel eignen sich bei günstiger Warmblütertoxizität zur Bekämpfung von pathogenen Endoparasiten die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp.,

Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria

spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp.. Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Strauße, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspension zur oralen oder dermalen Anwendung sowie zur Injektion; halbfeste Zubereitungen;

Formulierungen bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fordern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördemde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/ Caprinsäure-biglycerid, Triglyceridgemisch mit Pflanzenfettsäuren der Kettenlänge $C_{8-12}$ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der $C_8$/$C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:

Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;

ampholytische Tenside wie Di-Na-N-lauryl-β-iminodipropionat oder Lecithin;

anionaktive Tenside, wie Na-Laurylsulfat, Fettalkoholethersulfate, Mono/Dialkylpolyglykoletherorthophosphorsäureester-monoethanolaminsalz;

kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs-und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder linerares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffe, die gegen pathogene Endoparasiten wirken, vorliegen. Solche Wirkstoffe sind z.B. L-2,3,5,6-Tetrahydro-6-phenyl-imidazothiazol, Benzimidazolcarbamate, Pyrantel.

Anwendungsfertige Zubereitungen enthalten die Wirkstoffe in Konzentrationen von 10 ppm - 20 Gewichtsprozent, bevorzugt von 0,1-10 Gewichtsprozent.

Zubereitungen die vor Anwendung verdünnt werden, enthalten die Wirkstoffe in Konzentrationen von 0,5-90 Gew.-%, bevorzugt von 5 bis 50 Gewichtsprozent.

Im allgemeinen hat es sich als vorteilhaft erwiesen, Mengen der erfindungsgemäßen Mischung von etwa 10 bis etwa 100 mg Wirkstoff je kg Körpergewicht pro Tag zur Erzielung wirksamer Ergebnisse zu verabreichen. Bevorzugt sind 10 bis 50 mg Wirkstoffmischung je kg Körpergewicht.

In den Mitteln wird im allgemeinen ein Gewichtsverhältnis von Praziquantel bzw. Epsiprantel zu Depsipeptid wie 1 zu 1 bis 10 bevorzugt 1 zu 1 bis 2 ganz besonders bevorzugt 1 zu 1 eingehalten.

**Beispiel A**

In vivo Nematodentest

Ancylostoma caninum im Hund

Beagle-Welpen werden experimentell mit Hakenwürmern der Art Ancylostoma caninum infiziert. Zur Infektion wird den Hunden A. caninum oral als 250 $L_3$-Larven appliziert.

Nach Ablauf der Präpatenzzeit (oder beim Nachweis einer Larvenwirksamkeit während der Präpatenzzeit) werden die Wirkstoffe als reiner Wirkstoff in Gelatinekapseln oral (p.o.) appliziert.

Die Wirksamkeit wird nach 2 Methoden bestimmt.

1. Auszählung der mit dem Kot ausgeschiedenen Wurmeier vor und nach der Behandlung.

2. Die prozentuale Wirksamkeit im kritischen Test nach der Formel:

$$\text{\% Wirksamkeit} = \frac{\text{Abgehende Würmer nach Behandlung}}{\text{Abgehende Würmer nach Behandlung und verbliebene Würmer}} \times 100$$

In folgender Tabelle wird die Dosis (mg/kg) angegeben bei der 100 % Wirksamkeit nach beiden Bestimmungsmethoden erzielt wurde:

Tabelle A

| Wirkstoff | Dosis effectiva [mg/kg] |
|---|---|
| Cyclo-(MeLeu-D-Lac-MeLeu-D-PhLac-MeLeu-D-Lac-MeLeu-D-PhLac)- [PF 1022] | 1 |
| 2-(Cyclohexylcarbonyl)-1,2,3,6,7,11b-hexahydro-4H-pyrazino[2,1-a]iso-quinolin-4-on [Praziquantel] | 0 |

Tabelle A (fortgesetzt)

| Wirkstoff | Dosis effectiva [mg/kg] |
|---|---|
| 2-(Cyclohexylcarbonyl)-2,3,6,7,8,12b-hexahydro-pyrazino[2,1-a][2]benz-azepin-4(1H)-on [Epsiprantel] | 0 |
| Cyclo-(MeLeu-D-Lac-MeLeu-D-PhLac-MeLeu-D-Lac-MeLeu-D-PhLac)- [PF 1022] + 2-(Cyclohexylcarbonyl)-1,2,3,6,7,11b-hexahydro-4H-pyrazino[2,1-a]iso-quinolin-4-on [Praziquantel] (1:1) | 0,5 |
| Cyclo-(MeLeu-D-Lac-MeLeu-D-PhLac-MeLeu-D-Lac-MeLeu-D-PhLac)- [PF 1022] + 2-(Cyclohexylcarbonyl)-2,3,6,7,8,12b-hexahydro-pyrazino[2,1-a][2]benz-azepin-4(1H)-on [Epsiprantel] (1:1) | 0,5 |
| Cyclo(-MeLeu-D-Lac-MeLeu-D-p-MorPhLac-MeLeu-D-Lac-MeLeu-D-p-MorPhLac-) | 0,5 |
| Cyclo(-MeLeu-D-Lac-MeLeu-D-p-MorPhLac-MeLeu-D-Lac-MeLeu-D-p-MorPhLac-) + 2-(Cyclohexylcarbonyl)-1,2,3,6,7,11b-hexahydro-4H-pyrazino[2,1-a]iso-quinolin-4-on [Praziquantel] (1:1) | 0,25 |
| Cyclo(-MeLeu-D-Lac-MeLeu-D-p-MorPhLac-MeLeu-D-Lac-MeLeu-D-p-MorPhLac-) + 2-(Cyclohexylcarbonyl)-2,3,6,7,8,12b-hexahydro-pyrazino[2,1-a][2]benz-azepin-4(1H)-on [Epsiprantel] (1:1) | 0,25 |
| MeLeu: N-Methyl-L-leucin; D-Lac: D-Milchsäure; D-PhLac: D-β-Phenylmilchsäure; D-p-MorPhLac: D-β-p-Morpholino-phenylmilchsäure | |

**Beispiel B**

In vivo Nematodentest

Ancylostoma caninum in der Katze

Katzen werden experimentell mit Hakenwürmern der Art Ancylostoma tubaeforme infiziert. Zur Infektion wird den Katzen Ancylostoma tubaeforme oral als 250 $L_3$-Larven bzw. perkutan als 500 $L_3$-Larven appliziert.

Nach Ablauf der Präpatenzzeit (oder beim Nachweis einer Larvenwirksamkeit während der Präpatenzzeit) werden die Wirkstoffe als reiner Wirkstoff in Gelatinekapseln oral (p.o.) appliziert.

Die Wirksamkeit wird nach 2 Methoden bestimmt.

1. Auszählung der mit dem Kot ausgeschiedenen Wurmeier vor und nach der Behandlung.

2. Die prozentuale Wirksamkeit im kritischen Test nach der Formel:

$$\% \text{ Wirksamkeit} = \frac{\text{Abgehende Würmer nach Behandlung}}{\text{Abgehende Würmer nach Behandlung und verbliebene Würmer}} \times 100$$

In folgender Tabelle wird die Dosis (mg/kg) angegeben bei der 100 % Wirksamkeit nach beiden Bestimmungsmethoden erzielt wurde:

Tabelle B

| Wirkstoff | Dosis effectiva [mg/kg] |
|---|---|
| Cyclo-(MeLeu-D-Lac-MeLeu-D-PhLac-MeLeu-D-Lac-MeLeu-D-PhLac-) [PF 1022] | 1 |
| 2-(Cyclohexylcarbonyl)-1,2,3,6,7,11b-hexahydro-4H-pyrazino[2,1-a]iso-quinolin-4-on [Praziquantel] | 0 |
| 2-(Cyclohexylcarbonyl)-2,3,6,7,8,12b-hexahydro-pyrazino[2,1-a][2]benz-azepin-4(1H)-on [Epsiprantel] | 0 |

Tabelle B (fortgesetzt)

| Wirkstoff | Dosis effectiva [mg/kg] |
|---|---|
| Cyclo-(MeLeu-D-Lac-MeLeu-D-PhLac-MeLeu-D-Lac-MeLeu-D-PhLac)- [PF 1022] + 2-(Cyclohexylcarbonyl)-1,2,3,6,7,11b-hexahydro-4H-pyrazino[2,1-a]iso-quinolin-4-on [Praziquantel] (1:1) | 0,5 |
| Cyclo-(MeLeu-D-Lac-MeLeu-D-PhLac-MeLeu-D-Lac-MeLeu-D-PhLac)- [PF 1022] + 2-(Cyclohexylcarbonyl)-2,3,6,7,8,12b-hexahydro-pyrazino[2,1-a][2]benz-azepin-4(1H)-on [Epsiprantel] (1:1) | 0,5 |
| Cyclo(-MeLeu-D-Lac-MeLeu-D-p-MorPhLac-MeLeu-D-Lac-MeLeu-D-p-MorPhLac-) | 0,5 |
| Cyclo(-MeLeu-D-Lac-MeLeu-D-p-MorPhLac-MeLeu-D-Lac-MeLeu-D-p-MorPhLac-) + 2-(Cyclohexylcarbonyl)-1,2,3,6,7,11b-hexahydro-4H-pyrazino[2,1-a]iso-quinolin-4-on [Praziquantel] (1:1) | 0,25 |
| Cyclo(-MeLeu-D-Lac-MeLeu-D-p-MorPhLac-MeLeu-D-Lac-MeLeu-D-p-MorPhLac-) + 2-(Cyclohexylcarbonyl)-2,3,6,7,8,12b-hexahydro-pyrazino[2,1-a][2]benz-azepin-4(1H)-on [Epsiprantel] (1:1) | 0,25 |

MeLeu: N-Methyl-L-leucin; D-Lac: D-Milchsäure; D-PhLac: D-β-Phenylmilchsäure; D-p-MorPhLac: D-β-p-Morpholino-phenylmilchsäure

Beispiele für die Herstellung der cyclischen Depsipeptide mit 24 Ringatomen:

1. Herstellung der Verbindungen der Formel (Ia).
   Zu einer Lösung der Verbindung der Formel IIc (0,104 mmol) und Hünig-Base (0,258 mmol) in Dichlormethan (100 ml) wurde bei 0°C BOP-Cl (0,124 mmol) zugegeben und 24 h bei Raumtemperatur nachgerührt. Nach dieser Zeit wurden dieselben Mengen BOP-Cl und Base zugesetzt und weitere 24 h gerührt. Die Lösung wurde zweimal mit ges. Natriumhydrogencarbonatlösung gewaschen, über Natriumsulfat getrocknet und eingeengt. Der Rückstand wurde säulenchromatografisch mit dem Laufmittel Cyclohexan-Ethylacetat 2:1 gereinigt.

**Beispiele für die Herstellung der Verbindungen der Formel (IIc)**

Eine Lösung eines offentkettigen Octadepsipeptides der Formel (IIIa) (1,222 mmol) in Ethanol (50 ml) wurde in Gegenwart von $Pd(OH)_2/C$ (20 %; 200 mg) bis zur Beendigung der Wasserstoffaufnahme hydriert (ca. 2 h). Nach Abfiltrieren des Katalysators fiel reine Verbindung der Formel IIc an, die ohne zusätzliche Reinigung weiter umgesetzt wurde.

**Herstellung der Verbindungen der Formel (IIIa)**

In eine Lösung des tert.-Butylesters der Formel (IVa) (1.609 mmol) in Dichlormethan (40 ml) wurde bei 0°C 1,5 h HCl-Gas eingeleitet. Anschließend wurde auf Raumtemperatur aufgewärmt und 12h nachgerührt. Die Lösung wurde einrotiert und im Hochvakuum getrocknet. Der Rückstand wurde ohne weitere Reinigung umgesetzt.

**Herstellung der Verbindungen der Formel (IVa)**

Zu einer Lösung der Tetradepsipeptide der Formel (VIa) und (Va) je (2.52 mmol), in Dichlormethan (15 ml) wurde bei 0°C eine Lösung von Ethyldiisopropylamin (0,912 mmol) und BOP-C1 (0,438 mmol) zugegeben. Es wurde 1 h bei 0°C und 1,5 h bei Raumtemperatur nachgerübrt, mit 20 ml Dichlormethan verdünnt, zweimal mit wenig Wasser gewaschen, über $Na_2SO_4$ getrocknet und eingeengt. Der Rückstand wurde an Kieselgel mit dem Laufmittel Cyclohexan-t-BuOMe = 2 : 1 gereinigt.

**Herstellung der Verbindungen der Formel (Va)**

In eine Lösung des Tetradepsipeptids mit der Formel (VIIa) (2.848 mmol) in Dichlormethan (50 ml) wurde bei 0°C HCl-Gas 2 h eingeleitet.
Anschließend wurde 8 h bei Raumtemperatur nachgerührt, eingeengt und im Hochvakuum getrocknet. Der Rückstand wurde ohne weitere Reinigung eingesetzt.

**Herstellung der Verbindungen der Formel (VIa)**

Eine Lösung des Tetradepsipeptids mit der Formel (VIIa) (9.53 mmol) in Ethanol (37 ml) wurde mit $Pd(OH)_2$/C (20 %) (0,6 g) versetzt und ca. 3 h bei Raumtemperatur und Normaldruck hydriert. Das Reaktionsgemisch wurde filtriert, eingeengt und der Rückstand an Kieselgel mit dem Laufmittel t-BuOMe-Cyclohexan-Ethanol = 1:1:0,5 getrennt.

**Herstellung der Verbindungen der Formel (VIIa)**

Eine auf 0°C gekühlte Lösung des Didepsipeptids IXa (22,9 mmol) und des Didepsipeptids VIIIa (27,5 mmol) in Dichlormethan (80 ml) wurde mit Diisopropylethylamin (57,3 mmol) und BOP-Cl (29,8 mmol) versetzt, 1 h bei 0°C und 1 h bei Raumtemperatur gerührt. Nach Abfiltrieren des Niederschlages wurde die Lösung mit Dichlormethan verdünnt, dreimal mit wenig Wasser gewaschen, über Natriumsulfat getrocknet und eingeengt. Der Rückstand wurde an Kieselgel mit dem Laufmittel Cyclohexan-Ethylacetat = 15:1 getrennt.

**Patentansprüche**

**1.** Endoparasitizide Mittel, die Praziquantel oder Epsiprantel zusammen mit cyclischen Depsipeptiden der Formel

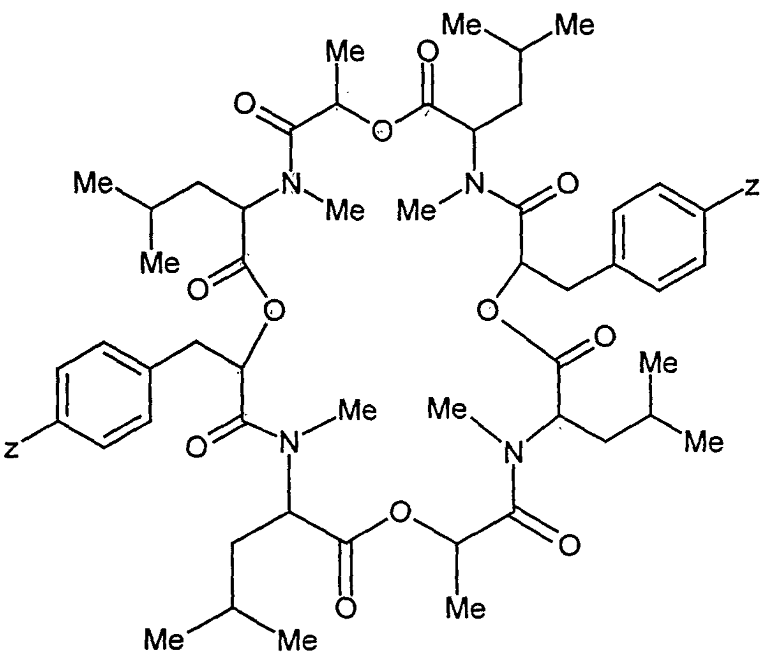

in welcher

Z für Wasserstoff (PF 1022A), N-Morpholino (Bis-morpholino-derivat, PF 1022-21), Nitro, Amino oder Dimethylamino steht,

bestehend aus Aminosäuren und Hydroxycarbonsäuren als Ringbausteinen und mit 24 Ringatomen enthalten.

**2.** Verwendung von Praziquantel oder Epsiprantel zusammen mit cyclischen Depsipeptiden der Formel

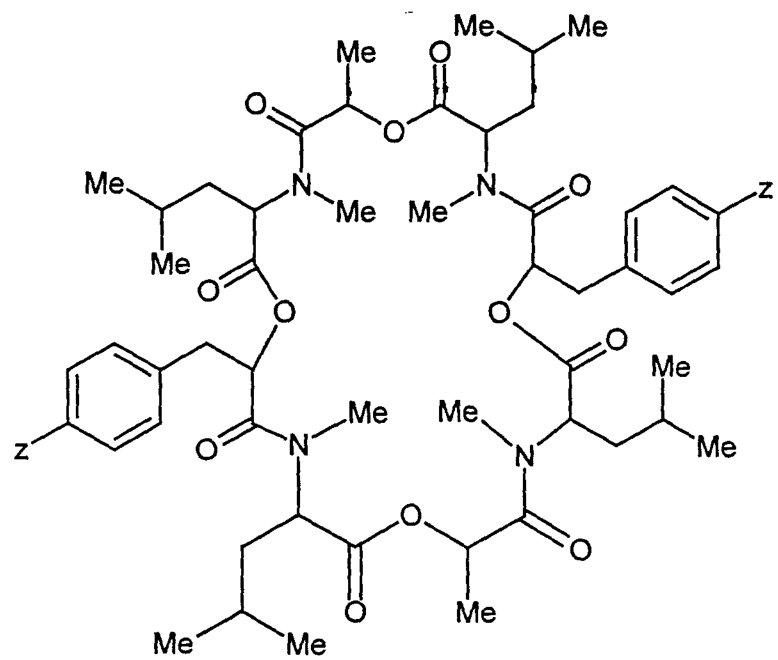

in welcher

Z für Wasserstoff (PF 1022A), N-Morpholino (Bis-morpholino-derivat, PF 1022-21), Nitro, Amino oder Dimethylamino steht,

bestehend aus Aminosäuren und Hydroxycarbonsäuren als Ringbausteinen und mit 24 Ringatomen zur Herstellung von endoparasitiziden Mitteln.

## Claims

1. Endoparasiticidal compositions comprising praziquantel or epsiprantel together with cyclic depsipeptides of the formula

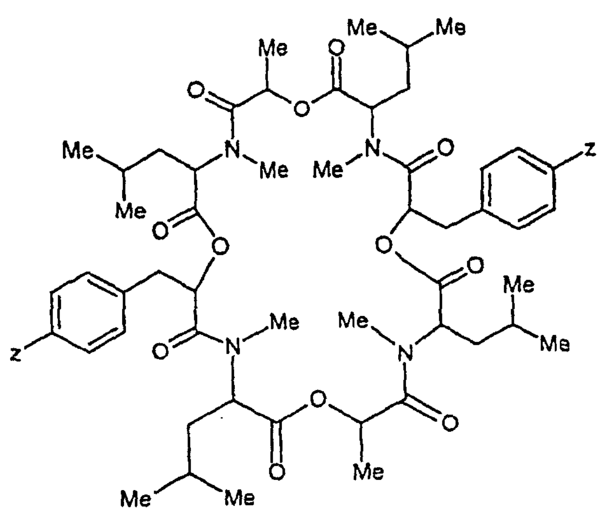

in which

Z represents hydrogen (PF 1022A), N-morpholino (bis-morpholino derivative, PF 1022-21), nitro, amino or dimethylamino,

composed of amino acids and hydroxycarboxylic acids as ring units and having 24 ring atoms.

**2.** Use of praziquantel or epsiprantel together with cyclic depsipeptides of the formula

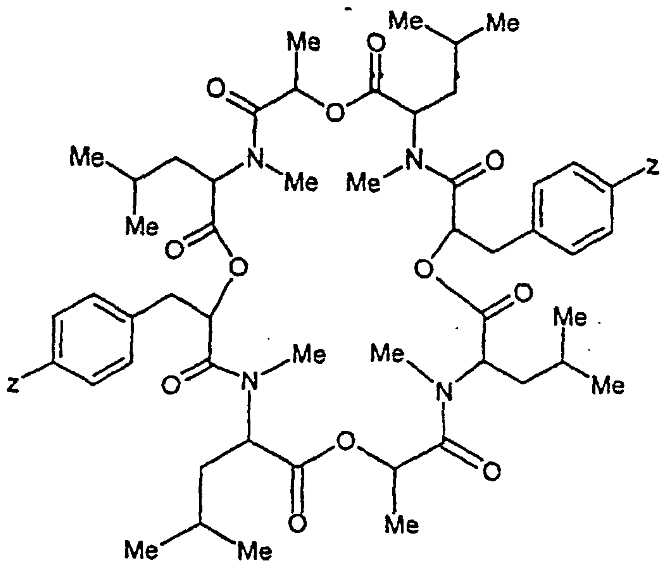

in which

Z represents hydrogen (PF 1022A), N-morpholino (bis-morpholino derivative, PF 1022-21), nitro, amino or dimethylamino,

composed of amino acids and hydroxycarboxylic acids as ring units and having 24 ring atoms, for the preparation of endoparasiticidal compositions.

## Revendications

**1.** Compositions endoparasiticides, qui contiennent du praziquantel ou de l'epsiprantel conjointement avec des depsipeptides cycliques de formule

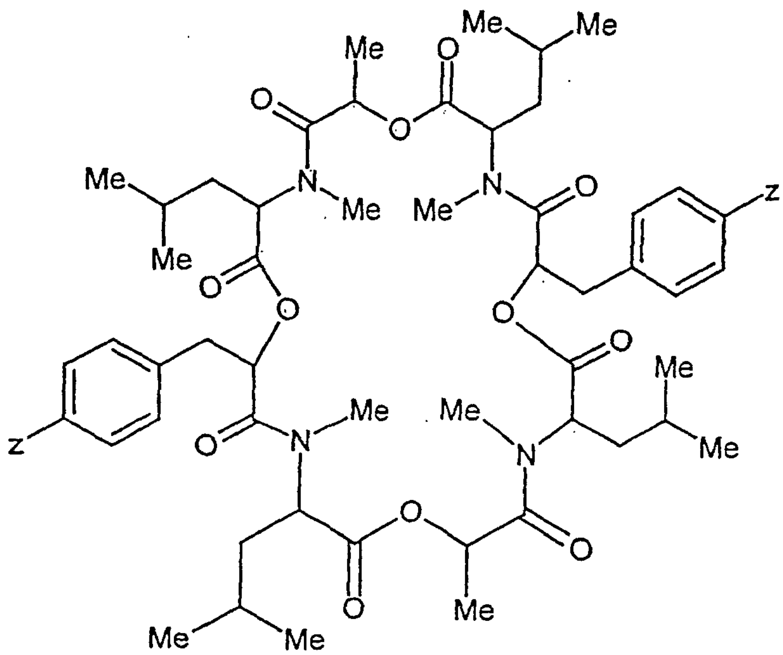

dans laquelle

Z représente l'hydrogène (PF 1022A), un groupe N-morpholino (dérivé bis-morpholino, PF 1022-21), nitro, amino ou diméthylamino,

constitués par des amino-acides et des acides hydroxycarboxyliques en tant que motifs formant le noyau et un noyau constitué de 24 atomes.

**2.** Utilisation du praziquantel ou de l'epsiprantel en association avec des depsipeptides cycliques de formule

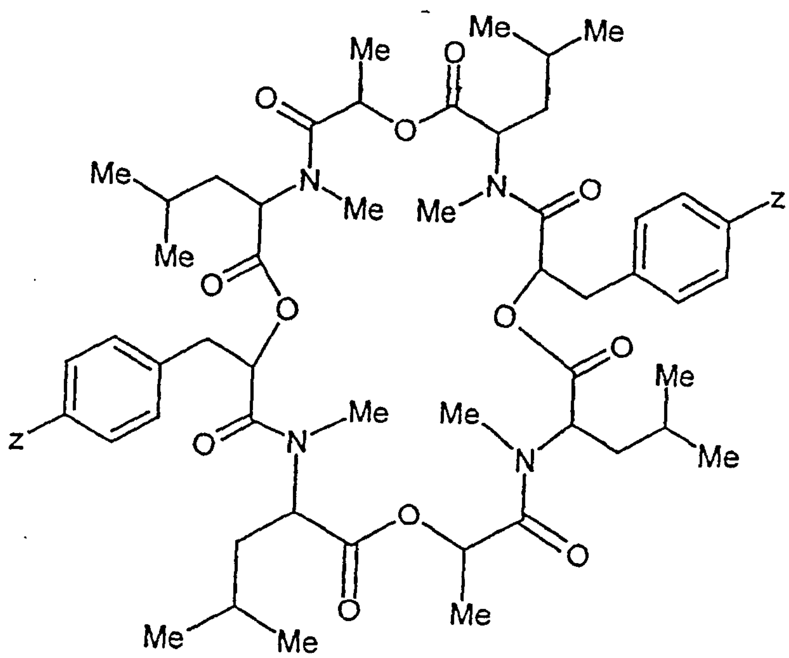

dans laquelle

Z représente l'hydrogène (PF 1022A),
un groupe N-morpholino (dérivé bis-morpholino, PF 1022-21), nitro, amino ou diméthylamino,

constitués par des amino-acides et des acides hydroxycarboxyliques en tant que motifs formant le noyau et un noyau de 24 atomes, pour la préparation de compositions endoparasiticides.